# EUROPEAN PATENT APPLICATION

(11) **EP 4 541 899 A1**
(43) Date of publication of application: **23.04.2025**
(21) Application number: 23823969.3
(22) Date of filing: 15.06.2023
(51) Int. Cl.: C12N 15/86, C12N 5/071, C12N 5/074, C12N 5/0775, C12N 5/09, C12N 15/861, C12N 15/864, C12N 15/867

(54) **METHOD FOR PRODUCING HIGH-TITER VIRAL VECTOR**

(30) Priority: 17.06.2022 JP 2022098128
(71) Applicant: NIPPON MEDICAL SCHOOL FOUNDATION, Bunkyo-ku Tokyo 113-8602 (JP); Kaneka Corporation, Osaka-shi, Osaka 530-8288 (JP)
(72) Inventor: YAMAZAKI Yoshiyuki, Tokyo 113-8602 (JP); MIYAGAWA Yoshitaka, Tokyo 113-8602 (JP); NITO Chikako, Tokyo 113-8602 (JP); HAYASHI Masahiro, Kobe-shi, Hyogo 650-0047 (JP); NAKAISHI Tomoyuki, Kobe-shi, Hyogo 650-0047 (JP)
(74) Representative: Hoffmann Eitle
(86) International application number: PCT/JP2023/022216
(87) International publication number: WO 2023/243679

(57) **Abstract**

This invention provides a viral vector with a higher infectious titer for application in gene and cell therapy. This invention relates to a method for producing a viral vector comprising, before introducing a plasmid containing a target gene into a virus-producing cell, a step of synchronizing a cell cycle of the virus-producing cell to the G2/M phase.

## Description

### Technical Field

The present invention relates to a method for producing a high-titer viral vector.

### Background Art

A viral vector infects a cell to express a particular target gene in a target cell or tissue. Since a viral vector makes use of an infecting ability of a virus, its gene transfection efficiency and expression efficiency are high, it enables gene transfection into a blood cell or primary cultured cell, which is difficult to perform by other gene transfection techniques, and it enables gene transfection *in vivo* into many animal species, such as rats and mice including humans. Accordingly, a viral vector is useful as a tool for genetic modification of a cell, gene and cell therapy for diseases, such as cancer and infectious diseases, and other applications.

**In** order to infect a cell with such viral vector with high efficiency, it is necessary to produce a virus with a high infectious titer. **In** particular, a retroviral vector is advantageous among various viral vectors because it is easily prepared and it can integrate a target gene into a chromosome to stably express the gene for a long period of time. However, a retroviral vector is insufficient in terms of an infectious titer. Methods for obtaining a viral vector with a high infectious titer have been disclosed. Patent Literature 1 discloses a method comprising culturing a retrovirus-producing cell in a medium containing hexamethylene bisacetamide, and Non-Patent Literature 1 discloses a method comprising treating a retrovirus-producing cell with trichostatin A (histone deacetylase). Concerning cell cycle regulation, Non-Patent Literature 2 reports that cell cycle synchronization to the G2/M phase before electroporation has led to an improvement in electrotransfection efficiency (eTE) and an expression level of a transgene.

### Citation List

### Patent Literature

Patent Literature 1: JP 2013-208107 A

### Non Patent Literature

Non Patent Literature 1: Tobias CA, et al., Improved recombinant retroviral titers utilizing trichostatin A, Biotechniques, Oct 2000, 29 (4): 884-90
Non Patent Literature 2: Cervia LD, et al., Enhancing Electrotransfection Efficiency through Improvement in Nuclear Entry of Plasmid DNA, Mol. Ther. Nucleic Acids, Jun 1, 2018; 11: 263-271

### Summary of Invention

### Technical Problem

The present invention provides a viral vector with a higher infectious titer for gene and cell therapy.

### Solution to Problem

The present inventors have conducted concentrated studies in order to dissolve the problems indicated above. As a result, they discovered that synchronization of a cell cycle of virus-producing cells to the G2/M phase followed by introduction of plasmids necessary for viral vector production has led to an remarkable improvement in infectious titers and, as a consequence, high-titer viral vectors could be produced. This has led to the completion of the present invention.

Specifically, the present invention includes the following.
(1) A method for producing a viral vector comprising, before introducing a plasmid containing a target gene into a virus-producing cell, a step of synchronizing a cell cycle of the virus-producing cell to the G2/M phase.
(2) The method according to (1), which comprises introducing a plasmid containing a gene necessary for viral particle formation and replication, simultaneously with the plasmid containing a target gene.
(3) The method according to (1) or (2), wherein the synchronization of a cell cycle to the G2/M phase is performed by culturing a virus-producing cell in a medium supplemented with an agent to arrest a cell cycle in the G2/M phase.
(4) The method according to (3), wherein the agent to arrest a cell cycle in the G2/M phase is Nocodazole.
(5) The method according to (1) or (2), wherein the synchronization of a cell cycle to the G2/M phase is performed by culturing a virus-producing cell in a medium supplemented with any one cell cycle-arresting agent selected from among an agent to arrest a cell cycle in the S phase, an agent to arrest a cell cycle in the G1 phase, and an agent to arrest a cell cycle in the G1/S phase and culturing the cell in a medium deprived of the agent to arrest a cell cycle for a given period of time to shift the cell cycle to the G2/M phase.
(6) The method according to (5), wherein the agent to arrest a cell cycle in the S phase is Aphidicolin.
(7) The method according to (1), wherein the viral vector is a retroviral vector, a lentiviral vector, an adenoviral vector, or an adeno-associated viral vector.
(8) The method according to (1), wherein the virus-producing cell is a mammal-derived somatic cell, a stem cell, a progenitor cell, or a cancer cell.
(9) The method according to (8), wherein the stem cell is a somatic stem cell.
(10) The method according to (9), wherein the somatic stem cell is a mesenchymal stem cell.
(11) A method for improving an infectious titer of a viral vector comprising, before introducing a plasmid containing a target gene into a virus-producing cell, a step of synchronizing a cell cycle of the virus-producing cell to the G2/M phase.

This patent application claims priority from Japanese Patent Application No. 2022-098128 filed on June 17, 2022, and includes part or all of the contents as disclosed in the description thereof.

### Advantageous Effects of Invention

The present invention provides a method for producing a viral vector with a high infectious titer. Therefore, the high-titer viral vector obtained according to the present invention is useful in clinical settings because of high therapeutic effects attained when it is used for gene and cell therapy of cancer or the like.

### Brief Description of Drawings

[Figure 1]

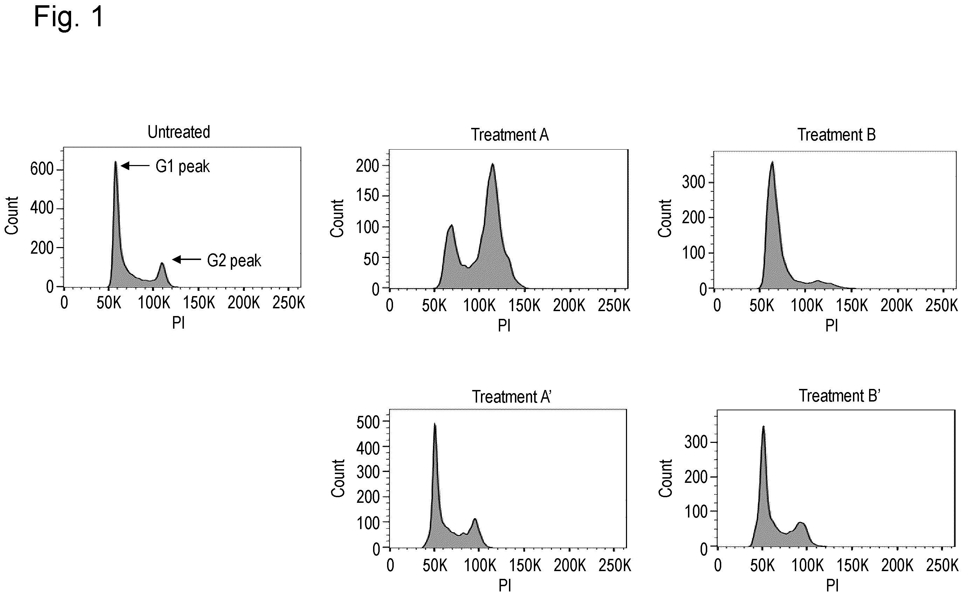

   Figure 1 shows histograms obtained by flow cytometric analysis of the population of untreated mesenchymal stem cells and the populations of mesenchymal stem cells subjected to Treatment A (the agent to arrest a cell cycle: 4 µg/mL Aphidicolin; the culture period after deprivation of the agent to arrest a cell cycle: 4 hours), Treatment A' (the same as Treatment A except for the addition of DMSO instead of the Aphidicolin solution in an amount equivalent to the amount thereof), Treatment B (the agent to arrest a cell cycle: 4 µg/mL Lovastatin; the culture period after deprivation of the agent to arrest a cell cycle: 4 hours), and Treatment B' (the same as Treatment B except for the addition of DMSO instead of the Lovastatin solution in an amount equivalent to the amount thereof).
[Figure 2]

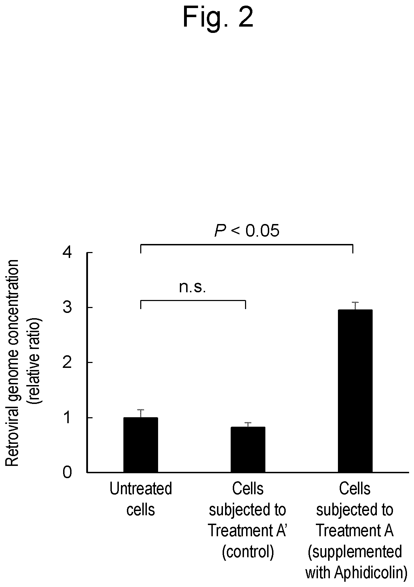

   Figure 2 shows the virus genome concentration (relative ratio) in the culture supernatants of untreated mesenchymal stem cells and the mesenchymal stem cells subjected to Treatment A (the agent to arrest a cell cycle: 4 µg/mL Aphidicolin; the culture period after deprivation of the agent to arrest a cell cycle: 4 hours) and Treatment A' (the same as Treatment A except for the addition of DMSO instead of the Aphidicolin solution in an amount equivalent to the amount thereof) (the value of the untreated cells is designated as 1). Statistical significance was detected by the Student's t-test.
[Figure 3]

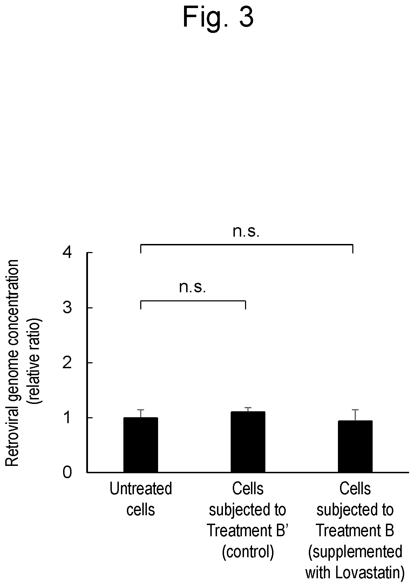

   Figure 3 shows the virus genome concentration (relative ratio) in the culture supernatants of untreated mesenchymal stem cells and the mesenchymal stem cells subjected to Treatment B (the agent to arrest a cell cycle: 4 µg/mL Lovastatin; the culture period after deprivation of the agent to arrest a cell cycle: 4 hours) and Treatment B' (the same as Treatment B except for the addition of DMSO instead of the Lovastatin solution in an amount equivalent to the amount thereof) (the value of the untreated cells is designated as 1). Statistical significance was detected by the Student's t-test.
[Figure 4]

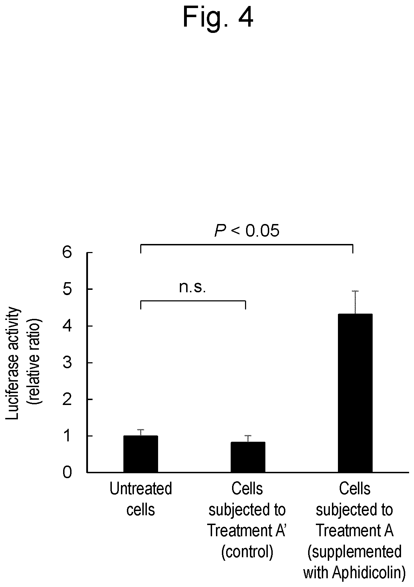

   Figure 4 shows luciferase activity (relative ratio) in HEK293 cells infected with the culture supernatants of untreated mesenchymal stem cells and the mesenchymal stem cells subjected to Treatment A (the agent to arrest a cell cycle: 4 µg/mL Aphidicolin; the culture period after deprivation of the agent to arrest a cell cycle: 4 hours) and Treatment A' (the same as Treatment A except for the addition of DMSO instead of the Aphidicolin solution in an amount equivalent to the amount thereof) (the value of the untreated cells is designated as 1). Statistical significance was detected by the Student's t-test.
[Figure 5]

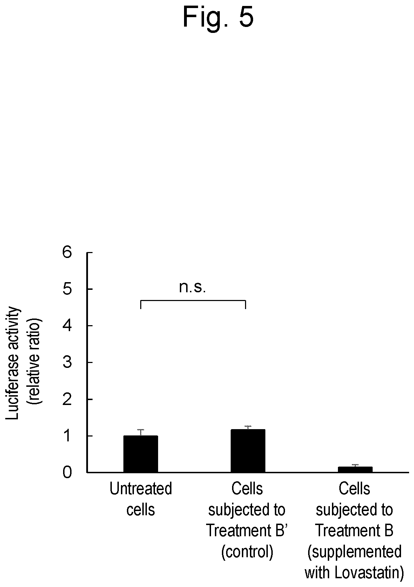

   Figure 5 shows luciferase activity (relative ratio) in HEK293 cells infected with the culture supernatants of untreated mesenchymal stem cells and the mesenchymal stem cells subjected to Treatment B (the agent to arrest a cell cycle: 4 µg/mL Lovastatin; the culture period after deprivation of the agent to arrest a cell cycle: 4 hours) and Treatment B' (the same as Treatment B except for the addition of DMSO instead of the Lovastatin solution in an amount equivalent to the amount thereof) (the value of the untreated cells is designated as 1). Statistical significance was detected by the Student's t-test.
[Figure 6]

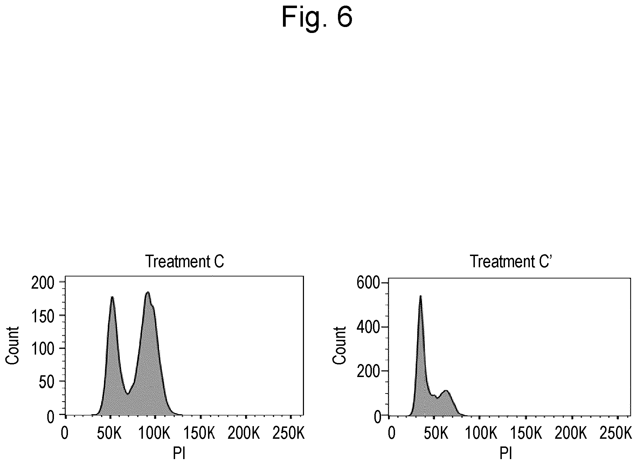

   Figure 6 shows histograms obtained by flow cytometric analysis of the populations of mesenchymal stem cells subjected to Treatment C (the agent to arrest a cell cycle: 1 µg/mL Aphidicolin; the culture period after deprivation of the agent to arrest a cell cycle: 4 hours) and Treatment C' (the same as Treatment C except for the addition of DMSO instead of the Aphidicolin solution in an amount equivalent to the amount thereof).
[Figure 7]

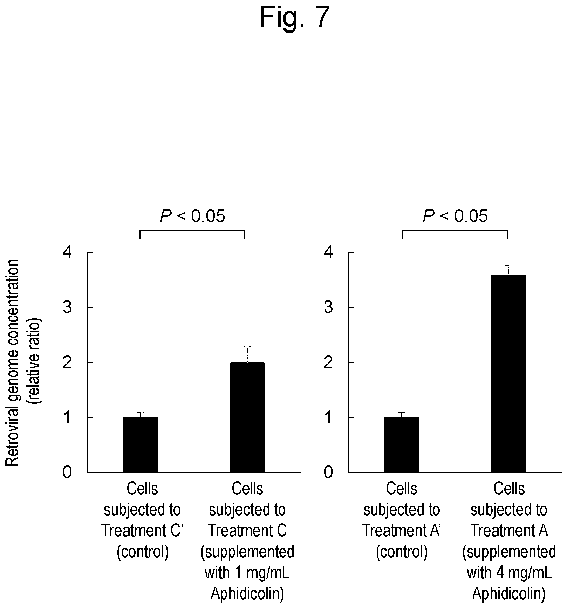

   Figure 7 shows the virus genome concentration (relative ratio) in the culture supernatants of the mesenchymal stem cells subjected to Treatment C (the agent to arrest a cell cycle: 1 µg/mL Aphidicolin; the culture period after deprivation of the agent to arrest a cell cycle: 4 hours), Treatment C' (the same as Treatment C except for the addition of DMSO instead of the Aphidicolin solution in an amount equivalent to the amount thereof), Treatment A (the agent to arrest a cell cycle: 4 µg/mL Aphidicolin; the culture period after deprivation of the agent to arrest a cell cycle: 4 hours), and Treatment A' (the same as Treatment A except for the addition of DMSO instead of the Aphidicolin solution in an amount equivalent to the amount thereof) (the values of the cells subjected to Treatment C' and of the cells subjected to Treatment A' are each designated as 1). Statistical significance was detected by the Student's t-test.
[Figure 8]

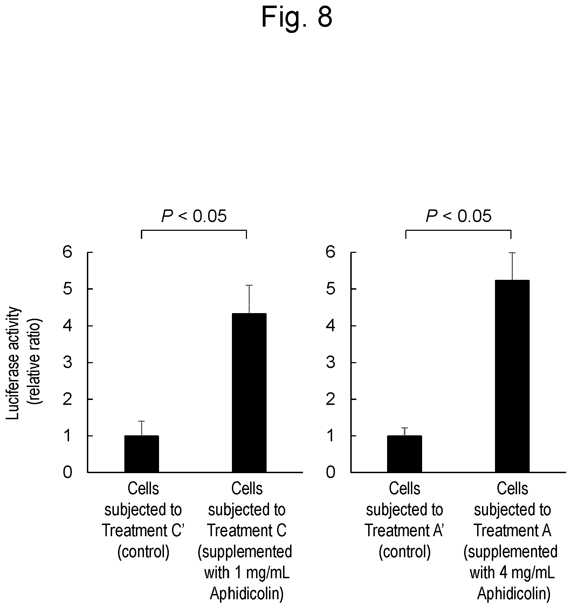

   Figure 8 shows luciferase activity (relative ratio) in HEK293 cells infected with the culture supernatants of the mesenchymal stem cells subjected to Treatment C (the agent to arrest a cell cycle: 1 µg/mL Aphidicolin; the culture period after deprivation of the agent to arrest a cell cycle: 4 hours), Treatment C' (the same as Treatment C except for the addition of DMSO instead of the Aphidicolin solution in an amount equivalent to the amount thereof), Treatment A (the agent to arrest a cell cycle: 4 µg/mL Aphidicolin; the culture period after deprivation of the agent to arrest a cell cycle: 4 hours), and Treatment A' (the same as Treatment A except for the addition of DMSO instead of the Aphidicolin solution in an amount equivalent to the amount thereof) (the values of the cells subjected to Treatment C' and of the cells subjected to Treatment A' are each designated as 1). Statistical significance was detected by the Student's t-test.
[Figure 9]

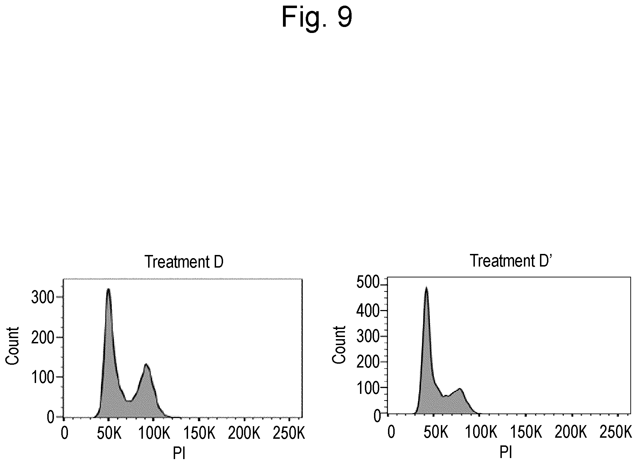

   Figure 9 shows histograms obtained by flow cytometric analysis of the populations of mesenchymal stem cells subjected to Treatment D (the agent to arrest a cell cycle: 0.1 µg/mL Nocodazole) and Treatment D' (the same as Treatment D except for the addition of DMSO instead of Nocodazole in an amount equivalent to the amount thereof).
[Figure 10]

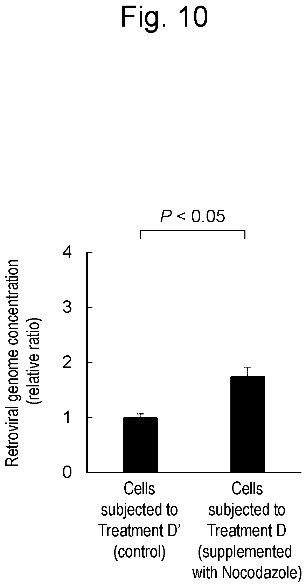

   Figure 10 shows the virus genome concentration (relative ratio) in the culture supernatants of the mesenchymal stem cells subjected to Treatment D (the agent to arrest a cell cycle: 0.1 µg/mL Nocodazole) and Treatment D' (the same as Treatment D except for the addition of DMSO instead of Nocodazole in an amount equivalent to the amount thereof) (the value of the cells subjected to Treatment D' is designated as 1). Statistical significance was detected by the Student's t-test.
[Figure 11]

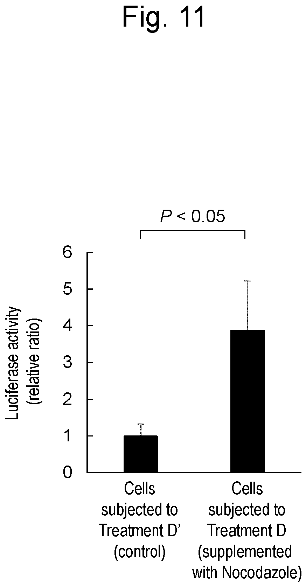

   Figure 11 shows luciferase activity (relative ratio) in HEK293 cells infected with the culture supernatants of the mesenchymal stem cells subjected to Treatment D (the agent to arrest a cell cycle: 0.1 µg/mL Nocodazole) and Treatment D' (the same as Treatment D except for the addition of DMSO instead of Nocodazole in an amount equivalent to the amount thereof) (the value of the cells subjected to Treatment D' is designated as 1). Statistical significance was detected by the Student's t-test.

### Description of Embodiments

### 1. Method for producing viral vector

The present invention provides a method for producing a high-titer viral vector by subjecting a virus-producing cell to a particular pretreatment (hereafter, such method may be abbreviated as "the production method of the present invention").

In an embodiment of the present invention, the production method of the present invention comprises, before introducing a plasmid containing a target gene into a virus-producing cell, a step of synchronizing a cell cycle of the virus-producing cell to the G2/M phase.

In another embodiment of the present invention, the production method of the present invention comprises, before introducing a plasmid containing a target gene and a plasmid containing genes necessary for viral particle formation and replication into a virus-producing cell, a step of synchronizing a cell cycle of the virus-producing cell to the G2/M phase.

### (Viral vector)

In the present invention, the term "viral vector" refers to a vector prepared by modifying a naturally-occurring virus via gene recombination to be capable of transfecting a target gene of interest into a target by virus infection.

A type of a viral vector used in the present invention is not particularly limited, provided that such viral vector is generally used for gene transfection. Examples thereof include a retroviral vector, a lentiviral vector, an adenoviral vector, and an adeno-associated viral (AAV) vector. A retroviral vector or lentiviral vector is particularly preferable because, after the virus infects the cell, the viral genome is integrated into a host chromosome, and the target gene integrated into the vector can be stably expressed for a long period of time.

### (Virus-producing cell)

In the present invention, the term "virus-producing cell" refers to a cell capable of virus production as a result of introduction of an element necessary for viral particle formation, so as to form and/or produce viral particles in the cell. Examples of the "virus-producing cell" used in the present invention include a wide variety of cells of mammals including humans, such as stem cells, somatic cells constituting organisms, progenitor cells, cancer cells, immortalized cells (cell lines) separated from organisms and stably maintained *ex vivo,* and cells separated from organisms and artificially modified. The origins of the cells are not particularly limited, provided that such cells are derived from mammals. Examples include humans, mice, rats, guinea pigs, hamsters, rabbits, dogs, cats, pigs, cows, and horses.

The "stem cells" encompass somatic stem cells and pluripotent stem cells. The term "somatic stem cell" refers to somatic stem cells existing in bone marrow, fat, fetal membrane (including an amniotic membrane), placenta, umbilical cord, dental pulp, blood (including cord blood), skin (epidermal, hypodermal, and subcutaneous tissues), hair follicle, brain, nerve, liver, pancreas, kidney, muscle, and other tissues. Examples thereof include mesenchymal stem cells, hematopoietic stem cells, vascular endothelial stem cells, hepatic stem cells, neural stem cells, pancreatic stem cells, intestinal stem cells, and germline stem cells. Mesenchymal stem cells (MSCs) are particularly preferable. Tissues from which mesenchymal stem cells are to be collected are not particularly limited. For example, MSCs may be collected from bone marrow, fat, fetal membrane (including an amniotic membrane), placenta, umbilical cord, and dental pulp tissues. Mesenchymal stem cells derived from the amniotic membrane are more preferable. The term "pluripotent stem cells" refers to stem cells that have capability of differentiating (pluripotency) into all cells existing in organisms derived from the ectoderm, the mesoderm, and the endoderm and have proliferative capacities. Examples of pluripotent stem cells include induced pluripotent stem cells (iPS cells), embryonic stem cells (ES cells), embryonic stem cells derived from cloned embryos obtained by nuclear transfer (ntES cells), multipotent germline stem cells (mGS cells), germline stem cells (GS cells), multipotent adult progenitor cells (MAPC cells), and multilineage-differentiating stress-enduring cells (MUSE cells), with iPS cells and ES cells being preferable.

The term "somatic cells constituting organisms" refers to cells that are collected from any tissues, systems, and organs, such as skin, muscles, bones, cartilages, connective tissues, blood vessels, blood (including cord blood), bone marrows, heart, eyes, brain, nerves, thymic gland, spleen, adrenal gland, lung, pancreas, liver, stomach, small intestine, large intestine, bladder, prostate gland, spermary, ovary, and uterus. Examples of somatic cells constituting organisms include fibroblasts, bone marrow cells, blood erythrocytes, blood granulocytes (e.g., neutrophils, acidophils, and basophils), monocytes, lymphocytes (T cells, B cells, and NK cells), platelets, macrophages, dendritic cells, bone cells, osteoblasts, cartilage cells, epidermal cells, corneocytes (keratinocytes), adipocytes, mesenchymal cells, epidermic cells, endothelial cells, hepatic parenchymal cells, cumulus cells, glial cells, neurons, oligodendrocytes, microglia, macroglia (astrocytes), cardiac cells, muscle cells, and pancreatic β-cells.

The term "progenitor cells" refers to cells in the middle of differentiation from the stem cells into particular somatic cells. Examples thereof include hematopoietic progenitor cells, myeloid progenitor cells (e.g., erythroblasts, myeloblasts, promyelocytes, and blood megakaryocytes), and lymphoid progenitor cells (e.g., monoblasts and lymphoblasts).

The term "cancer cells" refers to cells that are derived from the somatic cells and have acquired infinite proliferative capacities.

The term "cell lines" refers to cells that have acquired infinite proliferative capacities as a result of artificial operation *ex vivo.*

### (Synchronization of cell cycle to the G2/M phase)

The production method of the present invention comprises synchronizing a cell cycle to the G2/M phase before introducing a plasmid containing a foreign gene into the virus-producing cell. "Synchronization of a cell cycle" is a process by which cells at different phases of the cell cycle are brought to the same phase.

Synchronization of a cell cycle can be typically performed by arresting cells to a particular phase of the cell cycle with the use of the agent to arrest a cell cycle. According to an embodiment of the present invention, therefore, synchronization of a cell cycle of virus-producing cells to the G2/M phase can be performed by culturing the virus-producing cells in a medium supplemented with the agent to arrest a cell cycle in the G2/M phase. Examples of the agents to arrest a cell cycle in the G2/M phase include Nocodazole, Tozasertib, Myriocin, CFM-4, NSC-95397, RO-3306, Rabusertib, Mitomycin C, Alsterpaullone, Latorunculin A, Latorunculin B, FQI1, Colchicine, PJ34 HCl, Cantharidin, BI 2536, Volasertib, Calyculin A, Okadaic acid, Carfilzomib, Genistin, Etoposide, SKF-96365, Evodiamine, Geldanamycin, Genistein, Herbimycin A, Streptzocin, Alisertib, Buparlisib, Ganetespib, 2-AAPA, Cytochalasin B, Griseofulvin, S-trityl-cysteine, Monastrol, vinblastine sulfate, vincristine sulfate, Paclitaxel, AZ3146, BO-264, Reversine, Forchlorfenuron, Podophyllotoxin, Demecolcine, Camptothecin, and 5-fluorouracil. Concentration of the agent to arrest a cell cycle in the G2/M phase to be added to a medium is not particularly limited, provided that the cell cycle can be synchronized to the G2/M phase. Such concentration can be adequately modified depending on types of agents to arrest a cell cycle in the G2/M phase and types of cells. Such concentration is generally 0.01 to 1 µg/mL, and it is preferably 0.1 to 0.5 µg/mL. While culture temperature is not particularly limited, for example, it is 30°C to 40°C, and preferably 35°C to 37°C. A culture period is, for example, 5 to 30 hours, and preferably 15 to 20 hours.

According to another embodiment of the present invention, synchronization of a cell cycle of virus-producing cells to the G2/M phase can be performed by synchronizing the virus-producing cells to any of the S phase, the G1 phase, or the G1/S phase in advance and shifting the phase to the G2/M phase. Synchronization to the S phase, the G1 phase, or the G1/S phase can be performed by culturing the virus-producing cells in a medium supplemented with an agent to arrest a cell cycle selected from among the agent to arrest a cell cycle in the S phase, the agent to arrest a cell cycle in the G1 phase, and the agent to arrest a cell cycle in the G1/S phase. Examples of agents to arrest a cell cycle in the S phase include Aphidicolin, Methotrexate, Gemcitabine HCl, Resveratrol, and thymidine with high concentration. Examples of agents to arrest a cell cycle in the G1 phase include PLX-4032, FK-506, cyclosporine, 2-deoxy-D-glucose, 10058-F4, Cyclopamine, CB-839, KT-5823, β-lapachone, MK-2206, Mirin, NGI-1, Palbociclib, Baicalein, NVP-BEZ235, Apicidin, CI-994, Simvastatin, Lovastatin, SL-01, Rapamycin, Wortmannin, SMI-4a, Tunicamycin, Niclosamide, Icilin, KN-93, p15INK4B, p16INK4A, p18INK4C, p19INK4D, p21CIP1, and p27KIP1. Examples of agents to arrest a cell cycle in the G1/S phase include Crizotinib, DRB, Thiostrepton, hydroxyurea, RK-682, Cytochalasin D, and 5,6-dichlorobenzimidazole 1-β-D-ribofuranoside.

Synchronization of a cell cycle to the S phase, the G1 phase, or the G1/S phase may be performed without the use of an agent to arrest a cell cycle. For example, synchronization may be performed by a technique, such as radiation irradiation, contact inhibition, serum starvation culture, or amino acid starvation culture. Radiation irradiation is a technique of irradiating a cell population with a radiation to selectively allow cells in a cell cycle with low radiosensitivity to survive and synchronizing the cell cycle of the entire cell population. Contact inhibition is a technique of arresting a cell cycle by culturing cells at high density with the utilization of properties such that proliferation is terminated upon close cell-to-cell contact. Serum starvation culture and amino acid starvation culture are techniques of arresting a cell cycle by culturing cells while reducing the amount of sera and amino acids contained in the medium or completely removing sera and amino acids from the medium.

The shift to the G2/M phase can be performed by culturing the cell population subjected to radiation irradiation, contact inhibition, serum starvation culture, or amino acid starvation culture or the cell population subjected to culture in the medium supplemented with the agent to arrest a cell cycle in the S phase, the agent to arrest a cell cycle in the G1 phase, or the agent to arrest a cell cycle in the G1/S phase in a common medium for a given period of time. The given period of time is not particularly limited, provided that a majority of cells in the S phase, the G1 phase, or the G1/S phase can be shifted to the G2/M phase. While the given period of time varies depending on, for example, cells or methods used, a person skilled in the art can select an adequate period of time. When the mesenchymal stem cells derived from the amniotic membranes are used, as described in the examples below, a majority of the cells arrested in the S phase and cultured for 4 hours was found to be shifted to the G2/M phase. In a preferable embodiment, accordingly, a period of cell culture at the time of the shift from the S phase to the G2/M phase is approximately 4 hours.

In the present invention, a medium that is generally used for animal cell culture and supplemented with the agent to arrest a cell cycle may be used for virus-producing cell culture. Specifically, a basal medium containing ingredients necessary for cell survival and growth (e.g., inorganic salts, carbohydrates, hormones, essential amino acids, non-essential amino acids, or vitamins) may be used. Examples thereof include Dulbecco's Modified Eagle Medium (D-MEM), Alpha Modification of Minimum Essential Medium Eagle (α-MEM), Minimum Essential Medium (MEM), RPMI 1640, Basal Medium Eagle (BME), Dulbecco's Modified Eagle Medium:Nutrient Mixture F-12 (D-MEM/F-12), Glasgow Minimum Essential Medium (Glasgow MEM), and a Hank's solution (Hank's balanced salt solution). The medium may be supplemented with, for example, albumin, fetal bovine serum (FBS), a human platelet lysate (hPL), cell growth factors (e.g., fibroblast growth factor (FGF), platelet-derived growth factor (PDGF), epithelial growth factor (EGF), hepatocyte growth factor (HGF), vascular endothelial growth factor (VEGF), and insulin), cytokines (e.g., interleukins, chemokines, interferons, colony-stimulating factors, and tumor necrosis factors), neurotrophic factors (e.g., nerve growth factor (NGF), brain-derived neurotrophic factor (BDNF), and glial cell-derived neurotrophic factor (GDNF)), and other physiologically active substances, such as amino acids (e.g., glycine, phenylalanine, lysine, aspartic acid, and glutamic acid), vitamins (e.g., biotin, pantothenic acid, and vitamin D), serum albumin, and antibiotics.

A culture vessel used for cell culture is not particularly limited, and examples thereof include a flask, a petri dish, a dish, a plate, chamber slides, a tube, a tray, a culture bag, and a roller bottle.

### (Target gene)

In the present invention, the target gene to be introduced into the virus-producing cell synchronized to the G2/M phase may be adequately selected in accordance with the application of the cell population transformed with the viral vector produced in the present invention. Preferable examples of the target gene include therapeutic genes for humans and marker genes used for evaluation of gene transfection efficiency or expression stability, such as genes encoding GFP (green fluorescent protein), β-galactosidase, and luciferase. The target gene may be, for example, inserted into a plasmid so as to be expressed under the control of an adequate promoter and used in that state. Alternatively, an enhancer sequence, a terminator sequence, or an intron sequence may be present in a plasmid.

### (Production of viral vector)

The viral vector of the present invention is typically produced by introducing (1) a plasmid containing a target gene and a gene capable of transcribing RNA sealed in a viral particle (a transfer vector plasmid) and (2) a plasmid containing a gene necessary for viral particle formation and replication (a packaging plasmid) into the virus-producing cells synchronized to the G2/M phase to prepare cells that have acquired virus-producing capabilities and collecting a target viral vector produced in the culture supernatant of the cells. In the cells into which (1) the transfer vector plasmid and (2) the packaging plasmid have been introduced, (2) the plasmid expresses a protein necessary for viral particle production, but the plasmid does not comprise the LTR sequence and a packaging signal. Accordingly, the gene encoding the virus-derived protein contained in (2) the plasmid is not packaged in the viral particle, but the target gene of (1) the plasmid is packaged in the viral particle. That is, the viral vector produced by the virus-producing cell comprises the target gene of interest packaged in the viral particle. While the viral vector comprises the target gene of interest, it does not comprise the gene encoding the virus-derived protein. Thus, the vector can infect the cells to introduce the target gene thereinto, and the viruses would not be replicated in the infected cells. According to the production method of the present invention, (1) the plasmid and (2) the plasmid may be introduced simultaneously, or (1) the plasmid may be introduced into the packaging cell comprising (2) the plasmid introduced thereinto.

The plasmid may be introduced into the virus-producing cell by any method for introducing DNA into an animal cell. For example, a method known to a person skilled in the art, such as electroporation, the calcium phosphate method, lipofection, or the DEAE-dextran method, may be employed.

When producing a retroviral vector, more specifically, a transfer vector plasmid, such as a plasmid comprising 5' LTR (long terminal repeat), a packaging signal (Ψ), 3' LTR, and the target gene, and a packaging plasmid, such as a plasmid comprising the gag gene, the pol gene, and the env gene independently or in combination, are introduced into the virus-producing cell synchronized to the G2/M phase, and culture is then performed. The gag gene encodes a viral particle-constituting protein, the pol gene encodes a reverse transcriptase, and the env gene encodes a virus envelop protein. A virus envelop protein is not particularly limited, provided that it can serve as a retrovirus envelope. For example, the envelop protein of the amphotropic retrovirus that infects a wide variety of cells including humans other than rodents, the envelop protein of the ecotropic retrovirus that selectively infects rodents, the mouse leukemia virus (MLV) envelop protein, the vesicular stomatitis virus G (VSV-G) protein envelop protein, the gibbon ape leukemia virus (GaLV) envelop protein, and the feline endogenous virus (RD-114) envelop protein may be used. As a virus-producing cell, a cell comprising the gag gene, the pol gene, and the env gene introduced thereinto and expressed therein (a packaging cell) may also be used. The term "packaging cell" refers to a cell comprising the virus genome lacking the Ψ region necessary to be enveloped into the viral particle introduced thereinto and expressing all the proteins necessary to constitute the viral particles therein.

When producing a lentiviral vector, a transfer vector plasmid, such as a plasmid comprising RRE (rev-responsive element), cPPT (central polypurine tract), WPRE (woodchuck hepatitis virus posttranscriptional regulatory element), and the target gene, and a packaging plasmid, such as a plasmid comprising the gag gene, the pol gene, the regulatory gene (tat, rev), the modified gene (vif, vpr, vpu, nef) plasmid, and the env gene, are introduced into the virus-producing cell synchronized to the G2/M phase and then cultured.

Culture of the virus-producing cells comprising the plasmids introduced thereinto can be performed under ordinary culture conditions. Culture temperature is not particularly limited, and it is, for example, 30°C to 40°C, and preferably 35°C to 37°C. CO₂ concentration is, for example, 1% to 10%, and preferably 5% to 6%. A culture period is 24 to 120 hours, and preferably 48 to 96 hours.

After culture, the supernatant is collected from the culture solution to obtain a viral vector. In the present invention, the viral vector is produced in the form of the supernatant, a filtrate obtained by filtering the supernatant, or a concentrated or purified product obtained by a conventional concentration or purification technique. The resultant is, for example, frozen by an adequate method and stored before use. The viral vector produced in the present invention may be prepared in the form of a pharmaceutical composition comprising a pharmacologically acceptable solvent.

In the present invention, the term "infectious titer" refers to efficiency (capability) to infect a particular cell, and the term "genome titer" refers to the amount of viral vectors. The infectious titer can be measured by, for example, infecting the target cell with the viral vector and detecting transgene expression. The genome titer can be measured by, for example, subjecting the viral vector RNA to real-time PCR.

### Examples

Hereafter, the present invention is described in greater detail with reference to the examples, although the present invention is not limited to these examples.

### (Example 1) Examination of conditions for G2/M phase synchronization

### (1) Synchronization of cell cycle of mesenchymal stem cells

### (Treatment A: the agent to arrest a cell cycle: Aphidicolin; the culture period after deprivation of the agent to arrest a cell cycle: 4 hours)

The separated and cultured mesenchymal stem cells derived from the amniotic membrane were cultured in a medium for mesenchymal stem cell culture (α-MEM containing 5% hPL (final concentration)) supplemented with Aphidicolin (final concentration: 4 µg/mL) at 37°C overnight, and the cultured cells were further cultured in the same medium deprived of the agent to arrest a cell cycle for an additional 4 hours (hereafter, α-MEM containing 5% hPL (final concentration) is referred to as the "medium").

### (Treatment A': a control of Treatment A)

As a control group, mesenchymal stem cells were cultured under the same culture conditions as in Treatment A except that DMSO was added to the medium instead of the Aphidicolin solution in an amount equivalent to the amount thereof.

### (Treatment B: the agent to arrest a cell cycle: Lovastatin; the culture period after deprivation of the agent to arrest a cell cycle: 4 hours)

The separated and cultured mesenchymal stem cells derived from the amniotic membrane were cultured in a medium supplemented with Lovastatin (final concentration: 4 µg/mL) at 37°C two nights, and the cultured cells were further cultured in the same medium deprived of the agent to arrest a cell cycle for an additional 4 hours.

### (Treatment B': a control of Treatment B)

As a control group, mesenchymal stem cells were cultured under the same culture conditions as in Treatment B except that DMSO was added to the medium instead of the Lovastatin solution in an amount equivalent to the amount thereof.

### (2) Analysis of cell cycle of mesenchymal stem cells

The cell populations subjected to treatments in (1) were analyzed in terms of the amount of DNA using a flow cytometer. The proportion of the cells synchronized to the G2/M phase in the cell population was determined in the manner described below.
(i) The results of measurement of the cell population stained with 50 µg/mL propidium iodide (PI) were shown by dot-plotting SSC on the vertical axis and FSC on the horizontal axis.
(ii) Gates were defined for the cell population corresponding to the mesenchymal stem cells, and the histograms each displaying the cell count on the vertical axis and the fluorescent intensity of PI on the horizontal axis were developed.
(iii) In the histograms developed in (ii), the data were fitted to the cell populations in the G0/G1 phase, the G2/M phase, and the S phase using Flowjo software.
(iv) The proportion of the cells at each cell cycle fitted in (iii) among all the cells included in the gates selected in (ii) was determined.

Figure 1 shows histograms obtained by flow cytometric analysis of the untreated cell population and the cell populations subjected to Treatment A, Treatment A', Treatment B, and Treatment B'.

In the cell population obtained by Treatment A (the agent to arrest a cell cycle: Aphidicolin; the culture period after deprivation of the agent to arrest a cell cycle: 4 hours), the proportion of the cells synchronized to the G2/M phase was increased at least 3 times compared with that of the untreated cell population or the cell population obtained by Treatment A' (control).

In the cell population obtained by Treatment B (the agent to arrest a cell cycle: Lovastatin; the culture period after deprivation of the agent to arrest a cell cycle: 4 hours), the proportion of the cells synchronized to the G2/M phase was decreased to 1/2 or lower compared with that of the untreated cell population or the cell population obtained by Treatment B'

### (control).

The comparison between Treatment A and Treatment B demonstrates that Aphidicolin is effective as the agent to arrest a cell cycle, so as to obtain the cell population synchronized to the G2/M phase.

### (Example 2) Production of retroviral vector with cells synchronized to G2/M phase

### (1) Introduction of viral gene into mesenchymal stem cells derived from amniotic membrane

A plasmid comprising the retroviral gag gene and pol gene, a plasmid comprising the retroviral envelop protein gene, and a retroviral vector comprising the retroviral packaging signal and the luciferase gene were introduced into untreated mesenchymal stem cells and the mesenchymal stem cells each subjected to Treatment A, Treatment A', Treatment B, and Treatment B' in Example 1 using the Neon^{®} transfection system (Thermo Fisher Scientific). The cells were cultured in the media for 4 days after introduction, and the culture supernatants containing the retroviral vectors secreted from the cells were then collected.

### (2) Virus infection using retroviral vector

### (Measurement of genome titer)

With the use of the culture supernatants containing the retroviral vectors collected in (1) as templates, real-time PCR was performed with the use of the primers 1 and 2 designed for the luciferase gene sequences.
Primer 1: tgaccgagaa ggagatcgtg (SEQ ID NO: 1)
Primer 2: gagaatctcg cggatcttgc (SEQ ID NO: 2)

One Step TB Green^{®} PrimeScript^{(™)} RT-PCR Kit II (Takara Bio Inc.) was used as a RT-PCR reaction solution (a mixed reaction solution containing an enzyme and a fluorescent dye), and Applied Biosystems^{®} 7500 Real-Time PCR System (Thermo Fisher Scientific) was used as a real-time PCR apparatus. A reaction cycle comprising 42°C for 5 minutes, 95°C for 10 seconds, and 95°C for 3 seconds followed by 60°C for 30 seconds was repeated 40 times (the standard conditions for the Applied Biosystems^{®} 7500 Real-Time PCR System were employed for the reaction conditions of melting curve analysis). At the same time, the serially- diluted samples with the known luciferase gene levels were used as templates to quantify the retroviral genome concentration in each template and determine the genome titers.

Figure 2 and Figure 3 each show the results of genome titer quantification. When the retrovirus collected from the culture supernatant of the mesenchymal stem cells synchronized to the G2/M phase by Treatment A was used as a template, the genome titer was increased approximately 3 times compared with that obtained when the retrovirus collected from the culture supernatant of untreated mesenchymal stem cells or the mesenchymal stem cells subjected to Treatment A' (control) was used as a template. That is, a statistically significant change was observed (Figure 2, P < 0.05). When the retrovirus collected from the culture supernatant of the mesenchymal stem cells subjected to Treatment B, which could not be synchronized to the G2/M phase, was used as a template, in contrast, the genome titer was equivalent to the genome titer attained when the retrovirus collected from the culture supernatant of untreated mesenchymal stem cells or the mesenchymal stem cells subjected to Treatment B' (control) was used as a template. That is, no statistically significant change was observed (Figure 3, n.s.).

### (Measurement of infectious titer)

HEK293 cells were seeded at approximately 10⁴ cells/well on a 96-well petri dish. On the following day, the culture supernatant containing retroviral vectors collected in (1) supplemented with polybrene (8 µg/mL) was added at 20 µl/well, and the HEK293 cells were incubated at 37°C for 16 hours to infect the HEK293 cells with the retrovirus. Subsequently, the infected HEK293 cells were cultured for an additional 4 days and then subjected to luciferase assay to quantify the luciferase expression levels in the cells. The Bright-Glo^{(™)} luciferase assay system (Promega) was used as a reagent for luciferase assay, and Wallac 1420 ARVO MX (PerkinElmer) was used as a plate reader.

Figure 4 and Figure 5 each show the results of the luciferase assay. The luciferase activity level in the HEK293 cells supplemented with the culture supernatant of the mesenchymal stem cells subjected to Treatment A was increased approximately 4 times compared with the luciferase activity level in the HEK293 cells supplemented with the culture supernatant of the untreated mesenchymal stem cells or the mesenchymal stem cells subjected to Treatment A' (control). That is, a statistically significant change was observed (Figure 4, P < 0.05). In contrast, the luciferase activity level in the HEK293 cells supplemented with the culture supernatant of the mesenchymal stem cells subjected to Treatment B was lower than the luciferase activity level in the HEK293 cells supplemented with the culture supernatant of the untreated mesenchymal stem cells or the mesenchymal stem cells subjected to Treatment B' (control) (Figure 5).

The above results demonstrate that, in the mesenchymal stem cells derived from the amniotic membranes, synchronization of the cell population to the G2/M phase would improve the retroviral genome titer and infectious titer.

### (Example 3) Influence of concentration of agent to arrest cell cycle

Treatment A that had yielded the cell population producing high-titer viral vectors under the synchronizing conditions to the G2/M phase performed in Example 2 was examined in the same manner except that the concentration of the agent to arrest a cell cycle was changed to 1/4.

### (Treatment C: the agent to arrest a cell cycle: Aphidicolin; the culture period after deprivation of the agent to arrest a cell cycle: 4 hours)

The separated and cultured mesenchymal stem cells derived from the amniotic membrane were cultured in a medium supplemented with Aphidicolin (final concentration: 1 µg/mL) at 37°C overnight, and the cultured cells were further cultured in the same medium deprived of the agent to arrest a cell cycle for an additional 4 hours.

### (Treatment C': a control of Treatment C)

As a control group, mesenchymal stem cells were cultured under the same culture conditions as in Treatment C except that DMSO was added to the medium instead of the Aphidicolin solution in an amount equivalent to the amount thereof.

Figure 6 shows histograms obtained by flow cytometric analysis of the cell populations subjected to Treatment C and Treatment C'.

In the cell population subjected to Treatment C, the proportion of the cells synchronized to the G2/M phase was increased at least 2 times compared with that of the cell population subjected to Treatment C' (control). In comparison with the cell population subjected to Treatment A in which the cells were treated with Aphidicolin at higher concentration, however, the proportion of the cells synchronized to the G2/M phase was decreased (Figure 6 and Figure 1).

**Figure** 7 shows the results of genome titer quantification performed in the same manner as in Example 2, and Figure 8 shows the results of the luciferase assay performed in the same manner as in Example 2. When the retrovirus collected from the culture supernatant of the mesenchymal stem cells synchronized to the G2/M phase by Treatment C was used as a template, the genome concentration was increased by approximately 2 times compared with that obtained when the retrovirus collected from the culture supernatant of the mesenchymal stem cells subjected to Treatment C' (control) was used as a template. That is, a statistically significant change was observed (Figure 7, P < 0.05). The luciferase activity level in the HEK293 cells supplemented with the culture supernatant of the mesenchymal stem cells subjected to Treatment C was increased approximately 4 times compared with the luciferase activity level in the HEK293 cells supplemented with the culture supernatant of the mesenchymal stem cells subjected to Treatment C' (control). That is, a statistically significant change was observed (Figure 8, P < 0.05). **In** contrast, an increase in the genome titer and the luciferase activity of the cells subjected to Treatment C relative to the cells subjected to Treatment C' (control) was lower than an increase in the genome titer and the luciferase activity of the cells subjected to Treatment A relative to the cells subjected to Treatment A' (control) (Figure 7 and Figure 8).

The comparison between Treatment A and Treatment C demonstrates the following. As a result of Treatment C in which the amount of the agent to arrest a cell cycle to be added was reduced, the proportion of the cells synchronized to the G2/M phase and the genome titer were decreased compared with those achieved by Treatment A, and substantially no difference was observed in an increase in the luciferase activity between Treatment A and Treatment C. As a result of synchronization to the G2/M phase, specifically, viruses with high infectious titers can be obtained regardless of the genome titer.

### (Example 4) Influence of agent to arrest cell cycle

Treatment C that had yielded the cell population producing high-titer viral vectors under the synchronizing conditions to the G2/M phase in Example 3 was examined in the same manner except that the type and the concentration of the agent to arrest a cell cycle were changed.

### (Treatment D: the agent to arrest a cell cycle: Nocodazole)

The separated and cultured mesenchymal stem cells derived from the amniotic membrane were cultured in a medium supplemented with Nocodazole (final concentration: 0.1 µg/mL) at 37°C overnight.

### (Treatment D': a control of Treatment D)

As a control group, mesenchymal stem cells were cultured under the same culture conditions as in Treatment D except that DMSO was added to the medium instead of the Nocodazole solution in an amount equivalent to the amount thereof.

Figure 9 shows histograms obtained by flow cytometric analysis of the cell populations subjected to Treatment D and Treatment D'.

In the cell population subjected to Treatment D, the proportion of the cells synchronized to the G2/M phase was increased at least 1.5 times compared with that of the cell population subjected to Treatment D' (control).

Figure 10 shows the results of genome titer quantification performed in the same manner as in Example 2, and Figure 11 shows the results of the luciferase assay performed in the same manner as in Example 2. When the retrovirus collected from the culture supernatant of the mesenchymal stem cells synchronized to the G2/M phase by Treatment D was used as a template, the genome concentration was increased approximately 1.7 times compared with that obtained when the retrovirus collected from the culture supernatant of the mesenchymal stem cells subjected to Treatment D' (control) was used as a template (Figure 10). The luciferase activity level in the HEK293 cells supplemented with the culture supernatant of the mesenchymal stem cells subjected to Treatment D was increased approximately 4 times compared with the luciferase activity level in the HEK293 cells supplemented with the culture supernatant of the mesenchymal stem cells subjected to Treatment D' (control). That is, a statistically significant change was observed (Figure 11, P < 0.05).

The results demonstrate the following. The cell population synchronized to the G2/M phase was obtained as a result of Treatment D performed while modifying the type of the agent to arrest a cell cycle. While the proportion of the cells synchronized to the G2/M phase attained by Treatment D was lower than that attained by Treatment C, substantially no change was observed in an increase in the luciferase activity. That is, viruses with high infectious titers can be obtained.

### Industrial Applicability

The present invention can be used in the field of production of a viral vector that can serve as a tool for gene and cell therapy, vaccines, or gene function analysis.

All publications, patents, and patent applications cited herein are incorporated herein by reference in their entirety.

## Claims

1. A method for producing a viral vector comprising, before introducing a plasmid containing a target gene into a virus-producing cell, a step of synchronizing a cell cycle of the virus-producing cell to the G2/M phase.

2. The method according to claim 1, which comprises introducing a plasmid containing a gene necessary for viral particle formation and replication, simultaneously with the plasmid containing a target gene.

3. The method according to claim 1 or 2, wherein the synchronization of a cell cycle to the G2/M phase is performed by culturing a virus-producing cell in a medium supplemented with an agent to arrest a cell cycle in the G2/M phase.

4. The method according to claim 3, wherein the agent to arrest a cell cycle in the G2/M phase is Nocodazole.

5. The method according to claim 1 or 2, wherein the synchronization of a cell cycle to the G2/M phase is performed by culturing a virus-producing cell in a medium supplemented with any one cell cycle-arresting agent selected from among an agent to arrest a cell cycle in the S phase, an agent to arrest a cell cycle in the G1 phase, and an agent to arrest a cell cycle in the G1/S phase and culturing the cell in a medium deprived of the agent to arrest a cell cycle for a given period of time to shift the cell cycle to the G2/M phase.

6. The method according to claim 5, wherein the agent to arrest a cell cycle in the S phase is Aphidicolin.

7. The method according to claim 1, wherein the viral vector is a retroviral vector, a lentiviral vector, an adenoviral vector, or an adeno-associated viral vector.

8. The method according to claim 1, wherein the virus-producing cell is a mammal-derived somatic cell, a stem cell, a progenitor cell, or a cancer cell.

9. The method according to claim 8, wherein the stem cell is a somatic stem cell.

10. The method according to claim 9, wherein the somatic stem cell is a mesenchymal stem cell.

11. A method for improving an infectious titer of a viral vector comprising, before introducing a plasmid containing a target gene into a virus-producing cell, a step of synchronizing a cell cycle of the virus-producing cell to the G2/M phase.
